# EUROPEAN PATENT SPECIFICATION

(11) **EP 3 191 451 B1**
(45) Date of publication and mention of the grant of the patent: **05.06.2019**
(21) Application number: 15840917.7
(22) Date of filing: 09.09.2015
(51) Int. Cl.: C07D 213/79, A61K 31/4418, A61P 9/10, A61P 11/00, A61P 17/06, A61P 29/00, A61P 35/00

(54) **4-SUBSTITUTED PYRIDINE-2,6-DICARBOXYLIC ACID DERIVATIVES AND METHOD OF PREPARING SAME**
4-SUBSTITUIERTE PYRIDIN-2,6-DICARBONSÄURE-DERIVATE UND VERFAHREN ZUR HERSTELLUNG DAVON
DÉRIVÉS D'ACIDE PYRIDINE-2,6-DICARBOXYLIQUE 4-SUBSTITUÉS ET LEUR PROCÉDÉ DE PRÉPARATION

(30) Priority: 09.09.2014 US 201462048203 P
(43) Date of publication of application: 19.07.2017
(73) Proprietor: Okinawa Institute of Science and Technology School Corporation, Kunigami-gun, Okinawa 904-0495 (JP)
(72) Inventor: CHOUTHAIWALE, Pandurang Vilasrao, Okinawa 904-0495 (JP); TANAKA, Fujie, Okinawa 904-0495 (JP)
(74) Representative: Plougmann Vingtoft a/s
(86) International application number: PCT/JP2015/004588
(87) International publication number: WO 2016/038890

(56) References cited:
- WO-A1-2014/058078
- WO-A2-2008/082487
- JP-A- 2004 196 732
- US-A1- 2008 167 443
- US-A1- 2013 331 378
- GHOSHAL,A. ET AL.: 'RHODIUM-CATALYZED ASYMMETRIC HYDROSILYLATION OF KETONES EMPLOYING A NEW LIGAND EMBODYING THE BIS(OXAZOLINYL)PYRIDINE MOIETY' SYNLETT no. 10, 2010, pages 1459 - 1462, XP055418177
- TEYSSOT,M-L. ET AL.: 'Compound 34' EUROPEAN JOURNAL OF ORGANIC CHEMISTRY vol. 2010, no. 18, 2010, pages 3507 - 3515, XP055042351
- DATABASE REGISTRY [Online] 05 September 2012 '2,6-Pyridinedicarboxylic acid, 4-(carboxymethyl)- 2,6-dimethyl ester', XP055418320 Retrieved from STN Database accession no. 1393530-89-6
- CARABATEAS,P.M. ET AL.: 'TETRAETHYL 4-(3-NITROPHENYL)-2,3,5,6-PYRIDINETETRACARB OXYLATE' JOURNAL OF HETEROCYCLIC CHEMISTRY vol. 13, no. 4, 1976, pages 927 - 928, XP055418102
- CHOUTHAIWALE,P.V. ET AL.: 'REACTIONS OF PYRUVATES: ORGANOCATALYTIC SYNTHESIS OF FUNCTIONALIZED DIHYDROPYRANS IN ONE POT AND FURTHER TRANSFORMATIONS TO FUNCTIONALIZED CARBOCYCLES AND HETEROCYCLES' CHEM.COMM. vol. 50, no. 94, 2014, pages 14881 - 14884, XP055418115

## Description

### Technical Field

The present invention relates to novel pyridine-2,6-dicarboxilic acids and derivatives thereof useful for therapy and/or prophylaxis in a mammal. In addition, the present invention also relates to methods for the preparation of pyridine-2,6-dicarboxilic acids and derivatives thereof that are concise and that may be performed under mild reaction conditions.

### Background Art

4-substituted pyridine-2,6-dicarboxilic acids and their derivatives are important as building blocks in the synthesis of bioactive and biofunctional molecules, probes for visualization of cells and molecules of interest, solid-support reagents, and other functional molecules (PTL 1 and NPL 1).

Dihydropyran is an important core structure as often found in bioactive natural products, pharmaceutical and intermediates thereof. Further, dihydro-2H-pyran derivatives can prepared from aldehyde and pyruvate by use of a catalyst, for example, such as β-proline-catalyzed reaction process. (PTL 2).

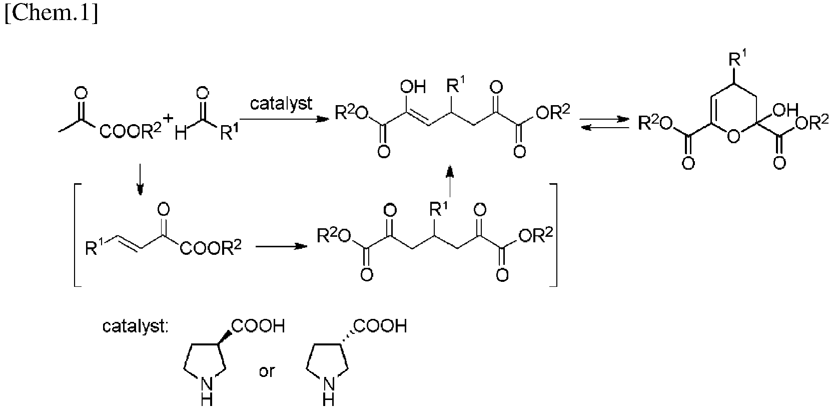

The dihydro-2H-pyran derivatives 1 may then be reacted with amines to form amino group substituted dihydropyran derivatives 11 and 12, nitrogen-containing heterocycles (including dihydrodiazepines 13 and 14), and quinoxalinone derivative 15 under mild conditions (NPL 2).

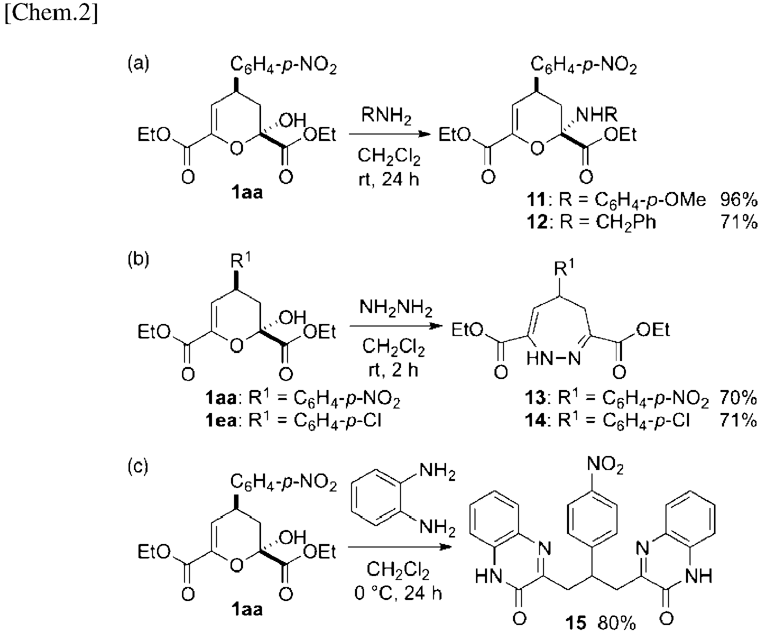

### Citation List

### Patent Literature

PTL 1: WO2005/021538
PTL 2: WO2014/058078

### Non Patent Literature

NPL 1: Atanu Ghoshal, etc., Synlett, 2010(10): 1459-1462.
NPL 2: Pandurang V. Chouthaiwale and Fujie Tanaka, Chem. Commun., 2014, 50, 14881-14884

### Summary of Invention

The present invention provides 4-substitued pyridine-2,6-dicarboxylic acid derivatives having the following structure: 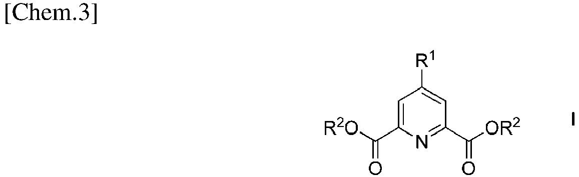 wherein R¹ is C₁C₁₈-alkyl, C₃-C₁₈-cycloalkyl, aryl, C₃-C₁₈-heterocycloalkyl or heteroaryl, which is optionally substituted with one or more substituent selected from the group consisting of C₁-C₁₈-alkyl, C₂-C₁₈-alkenyl, C₂-C₁₈-alkynyl, C₁-C₁₈-alkoxy, nitro, cyano, halogen, hydroxyl, carboxyl, halo-C₁-C₁₈-alkyl, halo-C₁-C₁₈-alkoxy and phenyl; and R² is C₁-C₁₈-alkyl or benzyl; or racemates, enantiomers, diastereomers, mixtures of the compound, or pharmaceutically acceptable salts thereof, except the specific compounds disclaimed in claim 1 of the appended set of claims.

The present description also provides methods for preparing 4-substitued pyridine-2,6-dicarboxylic acid derivatives, comprising reacting 3,4-dihydro-2H-pyran derivatives having the following structure: 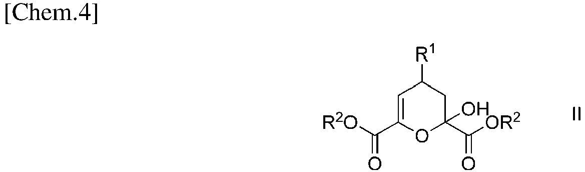 wherein R¹ and R² are as defined above, with NH₄OAc in a solvent.

In the method according to the description further comprising the preparation of 3,4-dihydro-2H-pyran derivatives: 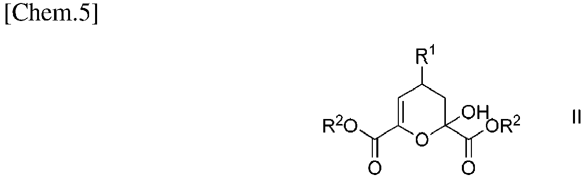 wherein R¹ and R² are as defined above, by aldol condensation-Michael addition-cyclization cascade reaction of pyruvates with aldehydes in a solvent by use of catalyst.

The present invention provides a pharmaceutical composition comprising the 4-substitued pyridine-2,6-dicarboxylic acid derivatives or racemates, enantiomers, diastereomers, mixtures of the compound, or pharmaceutically acceptable salts thereof or a pharmaceutically acceptable adjuvant.

### Technical Problem

The technical problem to be solved by the present invention is that of providing novel 4-substitued pyridine-2,6-dicarboxylic acid derivatives which may be used for bioactive and biofunctional molecules such as medicaments, probes, and ligands. In addition, the present invention also addresses the technical problem of providing a method for the preparation of 4-substituted pyridine-2,6-dicarboxylic acid derivatives in which may be concisely synthesized under mild reaction conditions.

### Solution to Problem

The present invention can provide novel 4-substitued pyridine-2,6-dicarboxylic acid derivatives. In the method according to the invention, pyridine-2,6-dicarboxylic acid derivatives bearing various substitutions at the 4-position may be concisely synthesized from 3,4-dihydro-2H-pyran derivatives and NH₄OAc in a solvent.

### Advantageous Effects of Invention

Novel 4-substitued pyridine-2,6-dicarboxylic acid derivatives of the present invention can be used for therapy and/or prophylaxis such as proliferative diseases. The method according to the invention can provide concisely synthesized pyridine-2,6-dicarboxylic acid derivatives bearing various substitutions at the 4-position under mild reaction conditions.

Further aspects and embodiments of the invention may become apparent to those skilled in the art from a review of the detailed description, the examples and the appended claims.

### Description of Embodiments

In the present disclosure, certain details are set forth such as specific quantities, concentrations, sizes, etc. so as to provide a thorough understanding of the various embodiments disclosed herein. However, it will be apparent to those skilled in the art that the present disclosure may be practiced without such specific details. In many cases, details concerning such considerations and the like have been omitted inasmuch as such details are not necessary to obtain a complete understanding of the present disclosure and are within the skill of persons of ordinary skill in the relevant art.

While most of the terms used herein will be recognizable to those of skill in the art, the following definitions are nevertheless put forth to aid in the understanding of the present disclosure. It should be understood, however, that when not explicitly defined, terms should be interpreted as adopting the meaning presently accepted by those of skill in the art.

The term "halogen" and "halo" are used interchangeably herein and denote fluoro, chloro, bromo, or iodo.

The term "alkyl" denotes a monovalent linear or branched saturated hydrocarbon group of 1 to 24 carbon atoms, in particular of 1 to 18 carbon atoms, more particular of 1 to 12 carbon atoms, further more particular of 1 to 8 carbon atoms for example, methyl, ethyl, n-propyl, isopropyl, n-butyl, iso-butyl, sec-butyl, and tert-butyl. The term "lower alkyl" refers an alkyl group of one to six carbon atoms, and includes, for example, methyl, ethyl, n-propyl and isopropyl.

The term "cycloalkyl", alone or in combination with other groups, denotes a monovalent saturated monocyclic or bicyclic hydrocarbon group of 3 to 18 ring carbon atoms, particularly a monovalent saturated monocyclic hydrocarbon group of 3 to 8 ring carbon atoms. Examples for monocyclic cycloalkyl are cyclopropyl, cyclobutanyl, cyclopentyl, cyclohexyl or cycloheptyl.

The term "alkoxy" denotes a group of the formula -O-R', wherein R' is an alkyl group. Examples of alkoxy group include methoxy, ethoxy, n-propoxy, isopropoxy, n-butoxy, isobutoxy and tert-butoxy. Particular alkoxy group include methoxy, ethoxy, n- propoxy and isopropoxy.

The term "halo-alkyl" denotes an alkyl group wherein at least one of the hydrogen atoms of the alkyl group has been replaced by same or different halogen atoms. Examples of haloalkyl include fluoromethyl, difluoromethyl, trifluoromethyl, 1,1,1-trifluoroethyl, 1,1,1-trifluoropropyl and pentafluoroethyl.

The term "halo-alkoxy" denotes an alkoxy group wherein at least one of the hydrogen atoms of the alkoxy group has been replaced by same or different halogen atoms. Examples of haloalkoxy include fluoromethoxy, difluoromethoxy, trifluoromethoxy, 1,1,1-trifluoroethoxy, 1,1,1-trifluoropropoxy, and pentafluoroethoxy.

The term "alkenyl" denotes a branched, unbranched or cyclic (e.g. in the case of C5 and C6) hydrocarbon group of 2 to 18, 2 to 12, or 2 to 8 carbon atoms, in some embodiments, carbon atoms containing at least one double bond, such as vinyl, allyl, octenyl, decenyl, dodecenyl, cyclohexenyl and the like. The term "lower alkenyl" refers an alkenyl group of two to six carbon atoms, and specifically includes vinyl and allyl. The term "cycloalkenyl" refers to cyclic alkenyl groups.

The term "alkynyl" denotes a branched or unbranched hydrocarbon group of 2 to 18, 2 to 12, or 2 to 8 carbon atoms, in some embodiments, carbon atoms containing at least one triple bond, such as acetylenyl, n-propynyl, n-butynyl, isobutynyl, octynyl, decynyl and the like. The term "lower alkynyl" refers an alkynyl group of two to six carbon atoms, and includes, for example, acetylenyl and propynyl. The term "cycloalkynyl" refers to cyclic alkynyl groups.

The term "hydroxyl", alone or in combination with other groups, refers to -OH.

The term "nitro", alone or in combination with other groups, refers to -NO₂.

The term "cyano", alone or in combination with other groups, refers to -CN.

The term "carboxyl", alone or in combination with other groups, refers to -COOH.

The term "aryl", alone or in combination with other groups, refers to an aromatic carbocyclic group comprising 6 to 14, preferably 6 to 10, carbon atoms and having at least one aromatic ring or multiple condensed rings in which at least one ring is aromatic. Examples of "aryl" include biphenyl, indanyl, naphthyl, phenyl (Ph) and the like. Preferred "aryl" is phenyl.

The term "heteroaryl", alone or in combination with other groups, refers to an aromatic carbocyclic group of having a single 4 to 8 membered ring or multiple condensed rings comprising 6 to 14, more preferably 6 to 10, ring atoms and containing 1, 2 or 3 heteroatoms individually selected from N, O and S, in particular N and O, in which group at least one heterocyclic ring is aromatic. Examples of "heteroaryl" include benzofuryl, benzoimidazolyl, benzooxazinyl, benzo thiazinyl, benzo thiazolyl, benzo thienyl, benzo triazolyl, furyl, imidazolyl, indazolyl, indolyl, isoquinolinyl, isothiazolyl, isoxazolyl, oxazolyl, pyrazinyl, pyrazolyl (pyrazyl), pyrazolo[1,5-a]pyridinyl, pyridazinyl, pyridinyl, pyrimidinyl, pyrrolyl, quinolinyl, tetrazolyl, thiazolyl, thienyl, triazolyl and the like. Preferred are 1H-pyrazolyl, furyl, isoxazolyl, oxazolyl, pyrazinyl, pyrazolyl, pyridazinyl, pyridinyl, pyridinyl-N-oxide and pyrimidinyl. More preferred heteroaryls are pyridinyl, pyrazolyl, pyrazinyl and pyrimidinyl.

The term "pharmaceutically acceptable salts" refers to those salts which retain the biological effectiveness and properties of the free bases or free acids, which are not biologically or otherwise undesirable. The salts are formed with inorganic acids such as hydrochloric acid, hydrobromic acid, sulfuric acid, nitric acid, phosphoric acid and the like, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein and the like. In addition these salts may be prepared by addition of an inorganic base or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins and the like. Particular pharmaceutically acceptable salts of compounds of formula (I) are the hydrochloride salts, methanesulfonic acid salts and citric acid salts.

The compounds of the present invention can contain several asymmetric centers and can be present in the form of optically pure enantiomers, mixtures of enantiomers such as, for example, racemates, optically pure diastereioisomers, mixtures of diastereoisomers, diastereoisomeric racemates or mixtures of diastereoisomeric racemates.

According to the Cahn-Ingold-Prelog Convention the asymmetric carbon atom can be of the "R" or "S" configuration.

The 4-substituted pyridine-2,6-dicarboxylic acid derivatives of the present invention may be represented by the following formula I: 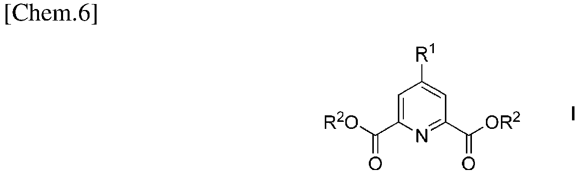 wherein R¹ is C₁-C₁₈-alkyl, C₃-C₁₈-cycloalkyl, aryl, C₃-C₁₈-heterocycloalkyl or heteroaryl, which is optionally substituted with one or more substituent selected from the group consisting of C₁-C₁₈-alkyl, C₂-C₁₈-alkenyl, C₂-C₁₈-alkynyl, C₁-C₁₈-alkoxy, nitro, cyano, halogen, hydroxyl, carboxyl, halo-C₁-C₁₈-alkyl, halo-C₁-C₁₈-alkoxy and phenyl; and R² is C₁-C₁₈-alkyl, preferably C₁-C₈-alkyl, more preferably C₁-C₄-alkyl, further more preferably C₂-C₄-alkyl or benzyl, except the specific compounds disclaimed in claim 1 of the appended set of claims. Compounds of formula I according to the invention include racemates, enantiomers, diastereomers, mixtures thereof, or pharmaceutically acceptable salts thereof.

In further embodiments of the compounds of formula I, R¹ is C₁-C₈-alkyl, C₃-C₈ - cycloalkyl, phenyl, naphthyl or 5- or 6-membered heteroaryl containing at least one hetero atom selected from nitrogen, oxygen and sulfur, which is optionally substituted with one or more substituents selected from the group consisting of C₁-C₈-alkyl, C₂-C₈ - alkenyl, C₂-C₈-alkynyl, C₁-C₈-alkoxy, nitro, cyano, halogen, hydroxyl, carboxyl, halo-C₁-C₈-alkyl, halo-C₁-C₈-alkoxy and phenyl; R² is C₁-C₈-alkyl, preferably C₁-C₄-alkyl, more preferably C₂-C₄-alkyl, or benzyl.

Additional embodiments of the invention include the compounds of formula I in which R¹ is C₁-C₈-alkyl substituted with one or more substituents selected from the group consisting of C₂-C₈-alkenyl, C₂-C₈-alkynyl, C₁-C₈-alkoxy, nitro, cyano, halogen, carboxyl, halo-C₁-C₈-alkyl, halo-C₁-C₈-alkoxy and phenyl; and R² is C₁-C₄-alkyl, preferably C₂-C₄-alkyl, or benzyl.

Additional embodiments of the invention include the compounds of formula I in which R¹ is C₃-C₈-cycloalkyl substituted with one or more substituents selected from the group C₂-C₈-alkenyl, C₂-C₈-alkynyl, C₁-C₈-alkoxy, nitro, cyano, halogen, carboxyl, halo-C₁-C₈-alkyl, halo-C₁-C₈-alkoxy and pheny; and R² is C₁-C₄-alkyl, preferably C₂-C₄ -alkyl, or benzyl.

Additional embodiments of the invention include the compounds of formula I in which R¹ is phenyl substituted with one or more substituents selected from the group methyl, ethyl, propyl, isopropyl, ethenyl, 2-propenyl, ethynyl, propargyl, trifluoromethyl, trifluoromethoxy, hydroxyl, carboxyl, nitro; and R² is C₁-C₄-alkyl, preferably C₂-C₄-alkyl, or benzyl.

Additional embodiments of the invention include the compounds of formula I in which R¹ is 5- or 6-membered heteroaryl containing at least one hetero atom selected from nitrogen, oxygen and sulfur substituted with one or more substituents selected from the group C₂-C₈-alkenyl, C₂-C₈-alkynyl, C₁-C₈-alkoxy, nitro, cyano, halogen, carboxyl, halo- C₁-C₈-alkyl, halo- C₁-C₈-alkoxy and phenyl; and R² is C₁-C₄-alkyl, preferably C₂-C₄-alkyl, or benzyl.

Additional embodiments of the invention include the compounds of formula I in which R¹ is 5- or 6-membered heterocyclyl containing at least one hetero atom selected from nitrogen, oxygen and sulfur substituted with one or more substituents selected from the group C₂-C₈-alkenyl, C₂-C₈-alkynyl, C₁-C₈-alkoxy, nitro, cyano, halogen, carboxyl, halo- C₁-C₈-alkyl, halo-C₁-C₈-alkoxy and phenyl; and R² is C₁-C₄-alkyl, preferably C₂-C₄-alkyl, or benzyl.

Additional particular embodiments of the invention include the compounds of formula I in which R² is methyl, ethyl, propyl, isopropyl, n-butyl, isobutyl, tert-butyl, or benzyl.

Additional particular embodiments of the invention include the compounds of formula I in which R² is C₂-C₄-alkyl, or benzyl.

Additional particular embodiments of the invention include the compounds of formula I in which R² is ethyl or benzyl.

Particular embodiments of the invention include the compound of formula I selected from the group consisting of:

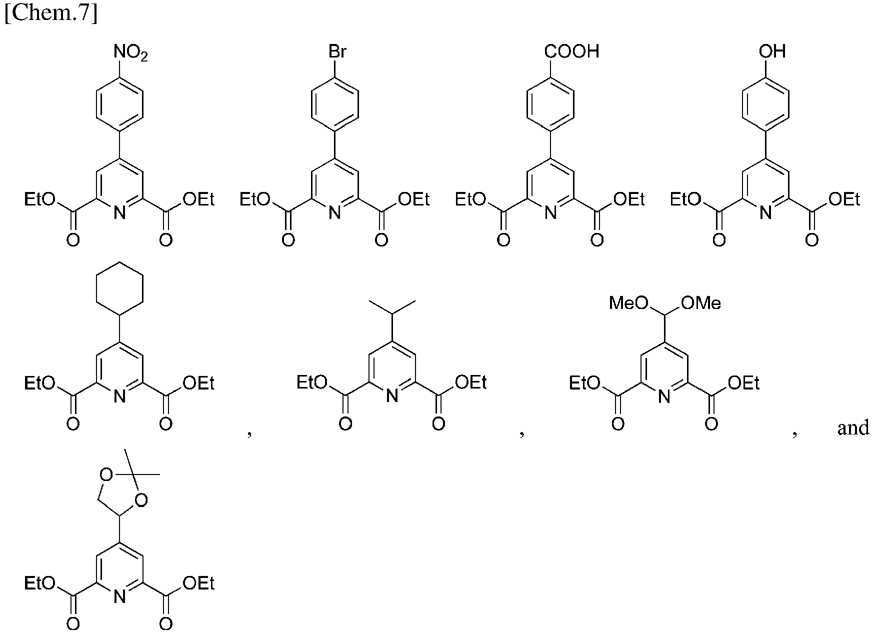

In addition to the compounds described above, the invention also relates to derivatives of these compounds, including racemates, enantiomers, diastereomers, mixtures thereof, or pharmaceutically acceptable salts thereof.

Another aspect of the present invention includes methods for preparing the compound of formula **I** as defined in claim 1 of the appended set of claims: 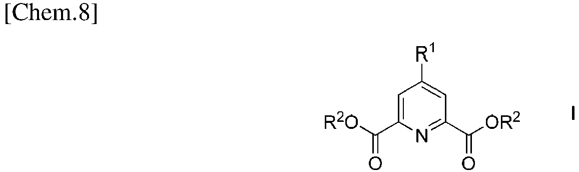 wherein R¹ and R² are as defined above, comprising reacting compound of formula II: 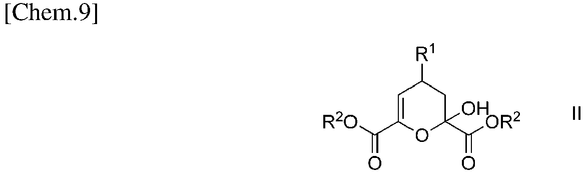 wherein R¹ and R² are as defined above, with NH₄OAc in a solvent.

In particular embodiments of the invention, R¹ is C₁-C₈-alkyl, preferably C₁-C₈-alkyl branched at the position next to the 4-position of the pyridine ring.

In particular embodiments of the invention, the molar ration of compound of formula II to NH₄OAc is 1 to 5, preferably 1 to 3, more preferably 1 to 2.2 .

In particular embodiments of the invention, the reaction can be carried out in a solvent, preferably in polar solvent such as CH₃CN, THF, dioxane, most preferably CH ₃CN.

In particular embodiments of the invention, the reaction can be carried out out at 4 to 40 °C, preferably at 10 to 30 °C, and more preferably at 15 to 28 °C.

Another aspects of the present invention further include methods for the preparation of the compound of formula II: 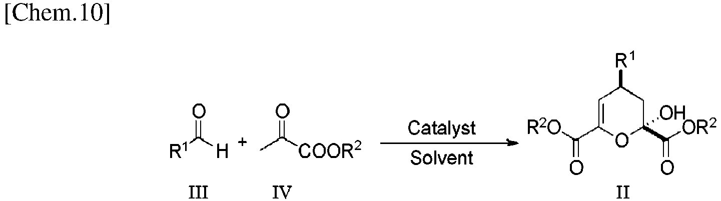 wherein R¹ and R² are as defined above.

For example, the compound of formula II may be prepared by adding (S)- -proline, (R)-β-proline, or (±)-β-proline to a solution of aldehyde and ethyl pyruvate (or methyl or benzyl pyruvate) in CH₃CN, at ambient temperature.

### Pharmacological Tests:

The compounds of formula I and their pharmaceutically acceptable salts possess valuable pharmacological properties. It has been found that the compounds of the present invention are associated with anti-cancer activity. The compounds were investigated in accordance with the test described below.

### Cytotoxicity:

K562 cells (American Type Culture Collection) were cultured in a culture medium containing 10% (v/v) fetal calf serum (FCS) (manufactures by Sigma) to logarithmic phase. The cells were seeded in 96-well microtiter plate at a density of 5000 cells/well in 100 µL cell culture medium and incubated at 37°C and 5% CO₂ in a humidified incubator overnight. The test compounds of various concentrations were added in a well at 10 fold concentration in 1/10 volume of medium without FCS. After 6-48 hrs incubation at 37°C in the CO₂ incubator, 10 uL solution of the viable cell counting reagent, Cell counting Kit-8 (5mmol/L WST-8, 0.2mmol/L 1-Methoxy PMS, 150mmol/L NaCl) (manufactured by Dojindo) was added to each well, and reacted for 1 to 4 hours in the CO₂ incubator. After the incubation, an absorbance of formazan, generated by reduction of WST-8, was determined at 450 nm using a microplate reader.

The assay readout is correlated with the viable cell numbers. Lower values correspond to high inhibition and greater values to low inhibition of the cell growth. To determine IC₅₀ values (i.e. the concentration inhibiting the cell growth by 50%) of the compounds of formula I, several assays were made with a range of concentrations chosen empirically to give low, high and intermediate inhibition of the growth and determined using curve fitting software.

The exemplified compounds according to formula I have an inhibitory activity in this assays (IC₅₀) particular less than 1000 µM, and more particularly less than 100 µM. The IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed.

The results are shown in Table 1.

### [Table 1]

**TABLE 1**

| Compound | Cytotoxic activity evaluated using K562 cells IC₅₀ (µM) |
|---|---|
| 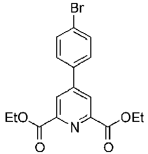 | 35 |
| 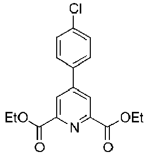 | 50 |
| 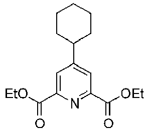 | 51 |

The compounds of formula I are useful in the treatment and prophylaxis of proliferative diseases.

The invention further relates to use of the compounds of formula I for the treatment and prophylaxis of proliferative diseases such as cancer, atherosclerosis, rheumatoid arthritis, psoriasis, idiopathic pulmonary fibrosis, scleroderma and cirrhosis of the liver.

The invention further relates to methods for the treatment and prophylaxis of proliferative diseases, in which the methods comprise administering an effective amount of the compounds of formula I to a patient in need.

### Pharmaceutical compositions

The compounds of formula I as well as their pharmaceutically acceptable salts can be used as medicaments, e.g. in the form of pharmaceutical preparations. The pharmaceutical preparations can be administered orally, e.g. in the form of tablets, coated tablets, dragees, hard and soft capsules, solutions, emulsions or suspensions. The administration can however, also be effected rectally, e.g. in the form of suppositories, or parenterally, e.g. in the form of injection solutions.

The compounds of formula I and their pharmaceutically acceptable salts can be processed with pharmaceutically inert, inorganic or organic excipients for the production of tablets, coated tablets, dragees and hard gelatin capsules. Lactose, corn starch or derivatives thereof, talc, stearic acid or its salts etc. can be used as such excipients e.g. for tablets, dragees and hard gelatin capsules.

Suitable excipients for soft gelatin capsules are e.g. vegetable oils, waxes, fats, semisolid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semi- liquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

The dosage can be varied within wide limits and will, of course, be adapted to the individual requirements in each particular case. In general, in the case of oral administration a daily dosage of about 10 to 1000 mg per person of a compound of general formula I should be appropriate, although the above upper limit may be exceeded when necessary.

### EXAMPLES

The invention is illustrated hereinafter by Examples, which have no limiting character. In case the preparative examples are obtained as a mixture of enantiomers and diastereomers, the pure enantiomers or diastereomers may be separated by methods described herein or by methods known to the person skilled in the art, such as chiral chromatography and crystallization.

### Reference Example 1

### (2S*,4S*)-Diethyl

### 2-hydroxy-4-(4-nitrophenyl)-3,4-dihydro-2H-pyran-2,6-dicarboxylate (1aa)

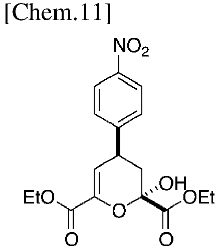

3,4-Dihydro-2H-pyrans of Example 1 (compound 1aa) was prepared as follows. To a solution of 4-nitorobenzaldehyde (1.0 mmol), and ethyl pyruvate (3.0mmol) in CH₃CN (1.0 mL), (S)-β-proline (23 mg, 0.2 mmol) was added at the room temperature (25 °C) and the mixture was stirred at the same temperature for 24 h. The mixture was poured into a saturated aqueous NH₄Cl solution (5 mL) and extracted with EtOAc. The organic layers were combined, dried over Na₂SO₄, filtered, concentrated, and purified by flash column chromatography (hexane/EtOAc) to afford the compound of Example 1 (3,4-dihydro-2H-pyran 1aa).

### <physical data>

**[Table 2]**

| |
|---|
| Flash column chromatography (hexane/EtOAc = 7:3); colorless solid. ¹H NMR (400 MHz, CDCl₃): δ 8.21 (d, *J* = 8.8 Hz, 2H), 7.45 (d, *J* = 8.8 Hz, 2H), 6.24 (dd, *J* = 2.4 Hz, 1.6, Hz, 1H), 4.60 (d, *J* = 2.0 Hz, 1H), 4.39-4.23 (m, 4H), 3.98 (ddd, *J* = 12.4 Hz, 6.0 Hz, 2.4 Hz, 1H), 2.22 (ddd, *J* = 13.2 Hz, 6.0 Hz, 1.6 Hz, 1H), 2.11 (ddd, *J* = 13.2 Hz, 12.4 Hz, 1.6 Hz, 1H), 1.33 (t, *J* = 7.2 Hz, 3H), 1.31 (t, *J* = 7.2 Hz, 3H). ¹³C NMR (100 MHz, CDCl₃): δ 168.7, 161.9, 149.9, 147.2, 141.9, 128.5, 124.1, 113.0, 94.3, 63.6, 61.6, 35.3, 34.7, 14.1, 13.9. ESI-HRMS: calcd for C₁₇H₂₀O₈N ([M+H]⁺) 366.1183, found 366.1190. |

### Reference Example 2

Diethyl 4-(4-chlorophenyl)-2-hydroxy-3,4-dihydro-2H-pyran-2,6-dicarboxylate (1ea)
Diethyl 4-(4-bromophenyl)-2-hydroxy-3,4-dihydro-2H-pyran-2,6-dicarboxylate (1fa)
4-(2,6-Bis(ethoxycarbonyl)-2-hydroxy-3,4-dihydro-2H-pyran-4-yl)benzoic acid (1ja)
Diethyl 2-hydroxy-4-(4-hydroxyphenyl)-3,4-dihydro-2H-pyran-2,6-dicarboxylate (11a)
Diethyl 4-(furan-2-yl)-2-hydroxy-3,4-dihydro-2H-pyran-2,6-dicarboxylate (1na)
Diethyl 4-cyclohexyl-2-hydroxy-3,4-dihydro-2H-pyran-2,6-dicarboxylate (1oa)
Diethyl 2-hydroxy-4-isopropyl-3,4-dihydro-2H-pyran-2,6-dicarboxylate (1pa)
Diethyl 4-(dimethoxymethyl)-2-hydroxy-3,4-dihydro-2H-pyran-2,6-dicarboxylate (1qa)
Diethyl 4-(2,2-dimethyl-1,3-dioxolan-4-yl)-2-hydroxy-3,4-dihydro-2H-pyran-2,6-dicarboxylat e (1ra)
Dibenzyl 2-hydroxy-4-phenyl-3,4-dihydro-2H-pyran-2,6-dicarboxylate (1gc)

3,4-Dihydro-2H-pyrans of Example 2 (1ea to 1ra, and 1gc) were prepared according to Example 1.

### Example 3

### Diethyl 4-(4-nitrophenyl)pyridine-2,6-dicarboxylate (16aa)

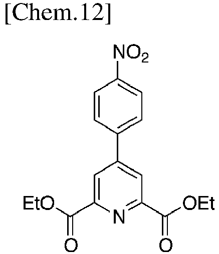

To a solution of 3,4-dihydro-2H-pyran 1aa (as a mixture with the corresponding linear form, 0.1 mmol) in CH₃CN (0.4 mL), ammonium acetate (16.9 mg, 0.22 mmol) was added at room temperature (25 °C), and the mixture was stirred at the same temperature for 24 h. The mixture was concentrated and purified by flash column chromatography to afford compound of Example 3 (4-substituted pyridine-2,6-dicarboxylic acid derivative 16aa).

### <physical data>

**[Table 3]**

| |
|---|
| Flash column chromatography (hexane/EtOAc = 3:2); pale yellow solid. ¹H NMR (400 MHz, CDCl₃): δ 8.51 (s, 2H), 8.39 (d, *J* = 8.8 Hz, 2H), 7.91 (d, *J* = 8.8 Hz, 2H) 4.53 (q, *J* = 7.1, Hz, 4H), 1.48 (t, *J* = 7.1, Hz, 6H). ¹³C NMR (100 MHz, CDCl₃): δ 164.4, 149.7, 148.7, 148.5, 142.6, 128.3, 125.6, 124.6, 62.6, 14.2. ESI-HRMS: calcd for C₁₇H₁₇O₆N₂ ([M+H]⁺) 345.1081, found 345.1081. |

### Reference Example 4

### Diethyl 4-(4-chlorophenyl)pyridine-2,6-dicarboxylate (16ea)

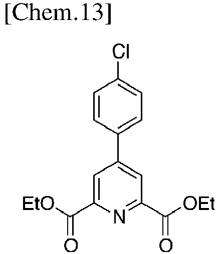

Compound of Example 4 (16ea) was prepared from diethyl 4-(4-chlorophenyl)-2-hydroxy-3,4-dihydro-2H-pyran-2,6-dicarboxylate (1ea) according to Example 3.

### <physical data>

**[Table 4]**

| |
|---|
| Flash column chromatography (hexane/EtOAc = 7:3); colorless solid. ¹H NMR (400 MHz, CDCl₃): δ 8.46 (s, 2H), 7.69 (d, *J* = 8.4 Hz, 2H), 7.51 (d, J= 8.8 Hz, 2H) 4.51 (q, J= 7.1 Hz, 4H), 1.47 (t, *J* = 7.1 Hz, 6H). ¹³C NMR (100 MHz, CDCl₃): δ 164.7, 149.7, 149.4, 136.4, 134.4, 129.6, 128.4, 125.2, 62.5, 14.2. ESI-HRMS: calcd for C₁₇H₁₇ClO₄N ([M+H]⁺) 334.0841, found 334.0836. |

### Example 5

### Diethyl 4-(4-bromophenyl)pyridine-2,6-dicarboxylate (16fa)

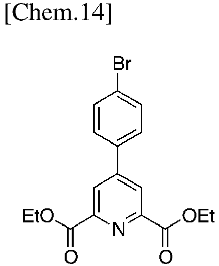

Compound of Example 5 (16fa) was prepared from diethyl 4-(4-bromophenyl)-2-hydroxy-3,4-dihydro-2H-pyran-2,6-dicarboxylate (1fa) ccording to Example 3.

### <physical data>

**[Table 5]**

| |
|---|
| Flash column chromatography (hexane/EtOAc = 7:3); colorless solid. ¹H NMR (400 MHz, CDCl₃): δ 8.46 (s, 2H), 7.67 (d, *J* = 8.8 Hz, 2H), 7.62 (d, *J* = 8.8 Hz, 2H) 4.51 (q, *J=* 7.1 Hz, 4H), 1.47 (t, *J* = 7.1 Hz, 6H). ¹³C NMR (100 MHz, CDCl₃): δ 164.7, 149.9, 149.4, 135.2, 132.2, 128.6, 125.2, 124.7, 62.5, 14.2. ESI-HRMS: calcd for C₁₇H₁₇BrO₄N ([M+H]⁺) 378.0335, found 378.0335. |

### Example 6

### 4-(2,6-Bis(ethoxycarbonyl)pyridin-4-yl)benzoic acid (16ja)

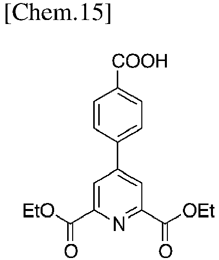

Compound of Example 6 (16ja) was prepared from 4-(2,6-Bis(ethoxycarbonyl)-2-hydroxy-3,4-dihydro-2H-pyran-4-yl)benzoic acid (1ja) according to Example 3.

### <physical data>

**[Table 6]**

| |
|---|
| Flash column chromatography (EtOAc/MeOH = 10:1); colorless solid. ¹H NMR (400 MHz, CD₃OD): δ 8.58 (s, 2H), 8.19 (d, *J* = 8.4 Hz, 2H), 7.93 (d, *J* = 8.4 Hz, 2H) 4.50 (q, *J* = 7.2 Hz, 4H), 1.46 (t, *J* = 7.2, Hz, 6H). ¹³C NMR (100 MHz, CD₃OD): δ 169.5, 165.8, 151.7, 150.7, 141.5, 131.7, 131.2, 128.4, 126.6, 63.4, 14.5. ESI-HRMS: calcd for C₁₈H₁₈O₆N ([M+H]⁺) 344.1129, found 344.1127. |

### Example 7

### Diethyl 4-(4-hydroxyphenyl)pyridine-2,6-dicarboxylate (161a)

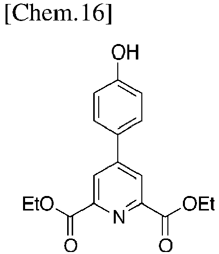

Compound of Example 7 (161a) was prepared from diethyl 2-hydroxy-4-(4-hydroxyphenyl)-3,4-dihydro-2H-pyran-2,6-dicarboxylate (11a) according to Example 3.

### <physical data>

**[Table 7]**

| |
|---|
| Flash column chromatography (hexane/EtOAc = 7:3); colorless solid. ¹H NMR (400 MHz, CD₃OD): δ 8.46 (s, 2H), 7.67 (d, *J* = 8.4 Hz, 2H), 7.00 (d, *J* = 8.4 Hz, 2H), 4.50 (q, *J* = 7.2 Hz, 4H), 1.46 (t, *J* = 7.2 Hz, 6H). ¹³C NMR (100 MHz, CD₃OD): δ 165.0, 157.9, 150.5, 149.1, 128.6, 128.4, 124.8, 116.4, 62.4, 14.2. ESI-HRMS: calcd for C₁₇H₁₈O₅N ([M+H]⁺) 316.1179, found 316.1179. |

### Reference Example 8

### Diethyl 4-(furan-2-yl)pyridine-2,6-dicarboxylate (6na)

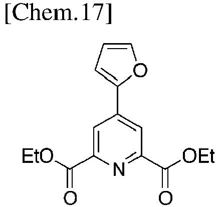

Compound of Example 8 (16na) was prepared from Diethyl 4-(furan-2-yl)-2-hydroxy-3,4-dihydro-2H-pyran-2,6-dicarboxylate (1na) according to Example 3.

### <physical data>

**[Table 8]**

| |
|---|
| Flash column chromatography (hexane/EtOAc = 7:3); colorless solid. ¹H NMR (400 MHz, CDCl₃): δ 8.45 (s, 2H), 7.61 (d, *J* = 1.6 Hz, 1H), 7.06 (dd, *J* = 3.6 Hz, 0.4 Hz, 1H), 6.57 (dd, *J* = 3.6 Hz, 1.6 Hz, 1H), 4.50 (q, *J* = 7.2 Hz, 4H), 1.47 (t, *J* = 7.2 Hz, 6H). ¹³C NMR (100 MHz, CDCl₃): δ 164.7, 149.9, 149.3, 144.9, 139.9, 121.5, 112.5, 110.8, 62.4, 14.2. ESI-HRMS: calcd for C₁₅H₁₆O₅N ([M+H]⁺) 290.1023, found 290.1027. |

### Example 9

### Diethyl 4-cyclohexylpyridine-2,6-dicarboxylate (16oa)

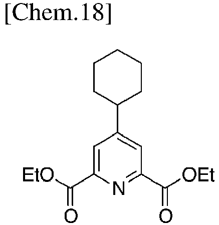

Compound of Example 9 (16oa) was prepared from diethyl 4-cyclohexyl-2-hydroxy-3,4-dihydro-2H-pyran-2,6-dicarboxylate (1oa) according to Example 3.

### <physical data>

**[Table 9]**

| |
|---|
| Flash column chromatography (hexane/EtOAc = 7:3); colorless solid. ¹H NMR (400 MHz, CDCl₃): δ 8.11 (s, 2H), 4.48 (q, *J* = 7.2 Hz, 4H), 2.70-2.63 (m, 1H), 1.96-1.86 (m, 4H), 1.82-1.76 (m, 1H), 1.45 (t, *J* = 7.2 Hz, 6H), 1.53-1.25 (m, 5H). ¹³C NMR (100 MHz, CDCl₃): δ 165.0, 159.5, 148.6, 126.5, 62.2, 43.9, 33.3, 26.3, 25.7, 14.2. ESI-HRMS: calcd for C₁₇H₂₄O₄N ([M+H]⁺) 306.1700, found 306.1702. |

### Example 10

### Diethyl 4-isopropylpyridine-2,6-dicarboxylate (16pa)

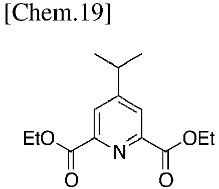

Compound of Example 10 (16pa) was prepared from Diethyl 2-hydroxy-4-isopropyl-3,4-dihydro-2H-pyran-2,6-dicarboxylate (1pa) according to Example 3.

### <physical data>

**[Table 10]**

| |
|---|
| Flash column chromatography (hexane/EtOAc = 7:3); colorless gum. ¹H NMR (400 MHz, CDCl₃): δ 8.12 (d, *J* = 0.8 Hz, 2H), 4.47 (dq, *J* = 7.2 Hz, 1.6 Hz, 4H), 3.10- 3.08 (m, 1H), 1.45 (dt, *J* = 7.2 Hz, 1.6 Hz, 6H), 1.31 (dd, *J* = 7.0 Hz, 1.6 Hz, 6H). ¹³C NMR (100 MHz, CDCl₃): δ 165.0, 160.4, 148.7, 126.1, 62.2, 33.7, 22.9, 14.2. ESI-HRMS: calcd for C₁₄H₂₀O₄N ([M+H]⁺) 266.1387, found 266.1388. |

### Example 11

### Diethyl 4-(dimethoxymethyl)pyridine-2,6-dicarboxylate (16qa)

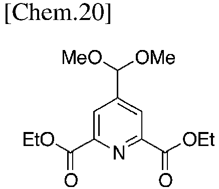

Compound of Example 11 (16qa) was prepared from diethyl 4-(dimethoxymethyl)-2-hydroxy-3,4-dihydro-2H-pyran-2,6-dicarboxylate (1qa) according to Example 3.

### <physical data>

**[Table 11]**

| |
|---|
| Flash column chromatography (hexane/EtOAc = 7:3); colorless solid. ¹H NMR (400 MHz, CDCl₃): δ 8.33 (s, 2H), 5.48 (s, 1H), 4.48 (q, *J* = 7.2, Hz, 4H), 3.34 (s, 6H), 1.44 (t, *J* = 7.2 Hz, 6H). ¹³C NMR (100 MHz, CDCl₃): δ 164.5, 149.8, 149.0, 125.9, 100.5, 62.3, 52.8, 14.2; ESI-HRMS: calcd for C₁₄H₂₀O₆N ([M+H]⁺) 298.1285, found 298.1287. |

### Example 12

### Diethyl 4-(2,2-dimethyl-1,3-dioxolan-4-yl)pyridine-2,6-dicarboxylate (16ra)

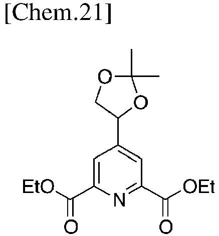

Compound of Example 12 (16ra) was prepared from diethyl 4-(2,2-dimethyl-1,3-dioxolan-4-yl)-2-hydroxy-3,4-dihydro-2H-pyran-2,6-dicarboxylat e (1ra) according to Example 3.

### <physical data>

**[Table 12]**

| |
|---|
| Flash column chromatography (hexane/EtOAc = 1:1); colorless gum. ¹H NMR (400 MHz, CDCl₃): δ 8.23 (s, 2H), 5.18 (t, *J* = 6.8 Hz, 1H) 4.48 (q, *J* = 7.2 Hz, 4H), 4.43 (dd, *J* = 8.4 Hz, 6.8, Hz, 1H), 3.72 (dd, *J* = 8.4 Hz, 7.2 Hz, 1H), 1.56 (s, 3H), 1.49 (s, 3H), 1.44 (t, *J* = 7.2 Hz, 6H). ¹³C NMR (100 MHz, CDCl₃): δ 164.5, 152.1, 149.0, 124.8, 110.9, 75.9, 70.7, 62.4, 26.3, 25.6, 14.2. ESI-HRMS: calcd for C₁₆H₂₂O₆N ([M+H]⁺) 324.1442, found 324.1440 |

### Industrial Applicability

The present invention relates to novel compounds of formula I wherein R¹ and R² are defined herein. The compounds of formula I are useful for making pharmaceutical compositions to treat proliferative diseases. The present invention also relates to concise methods of preparing compounds of formula I that may be performed under mild reaction conditions.

## Claims

1. A compound comprising the structure according to formula I, wherein 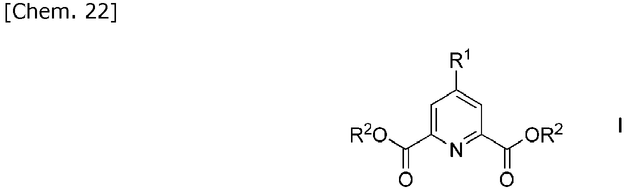
R¹ is C₁-C₁₈-alkyl, C₃-C₁₈-cycloalkyl, aryl, C₃-C₁₈-heterocycloalkyl or heteroaryl, which is optionally substituted with one or more substituent selected from the group consisting of C₁-C₁₈-alkyl, C₂-C₁₈-alkenyl, C₂-C₁₈-alkynyl, C₁-C₁₈-alkoxy, nitro, cyano, halogen, hydroxyl, carboxyl, halo-C₁-C₁₈-alkyl, halo-C₁-C₁₈-alkoxy and phenyl; and
R² is C₁-C₁₈-alkyl or benzyl;
or racemates, enantiomers, diastereomers, mixtures of the compound, or pharmaceutically acceptable salts thereof, except
Dimethyl 4-methylpyridine 2,6-dicarboxylate,
Dimethyl 4-(hydroxymethyl)-pyridine 2,6-dicarboxylate,
Dimethyl 4-ethylpyridine 2,6-dicarboxylate,
Dimethyl 4-isopropylpyridine 2,6-dicarboxylate,
Dimethyl 4-(hydroxyethyl)-pyridine 2,6-dicarboxylate,
Dimethyl 4-(chloromethyl)pyridine 2,6-dicarboxylate,
Dimethyl 4-(iodomethyl)pyridine 2,6-dicarboxylate,
Dimethyl 4-(bromomethyl)pyridine 2,6-dicarboxylate,
Dimethyl 4-(dimethoxymethyl)pyridine 2,6-dicarboxylate,
Dimethyl 4-(hydroxyethyl)-pyridine 2,6-dicarboxylate,
Dimethyl 4-phenyl-pyridine 2,6-dicarboxylate,
Dimethyl 4-(3-bromopropyl)-pyridine 2,6-dicarboxylate,
Dimethyl 4-(trifluoromethyl)-pyridine 2,6-dicarboxylate,
Dimethyl 4-(3-methylphenyl)-pyridine 2,6-dicarboxylate,
Dimethyl 4-cyclohexyl-pyridine 2,6-dicarboxylate,
Dimethyl 4-(4-methylphenyl)-pyridine 2,6-dicarboxylate,
Dimethyl 4-(4-hydroxyphenyl)-pyridine 2,6-dicarboxylate,
Dimethyl 4-(2-methylphenyl)-pyridine 2,6-dicarboxylate,
Dimethyl 4-(3-chlorophenyl)-pyridine 2,6-dicarboxylate,
Dimethyl 4-naphthalenyl-pyridine 2,6-dicarboxylate,
Dimethyl 4-(4-methoxylphenyl)-pyridine 2,6-dicarboxylate,
Dimethyl 4-(4-fluorophenyl)-pyridine 2,6-dicarboxylate,
Dimethyl 4-(4-nitrophenyl)-pyridine 2,6-dicarboxylate,
Dimethyl 4-(4-hydroxy-2-methoxylphenyl)-pyridine 2,6-dicarboxylate,
Dimethyl 4-(2,4-dimethoxylphenyl)-pyridine 2,6-dicarboxylate,
Dimethyl 4-[(4-octyloxyl)phenyl]-pyridine 2,6-dicarboxylate,
Dimethyl 4-benzo[b]thien-2-yl-pyridine 2,6-dicarboxylate,
Dimethyl 4-[(4-octadecyloxyl)phenyl]-pyridine 2,6-dicarboxylate,
Dimethyl 4-(1-methyl-1H-indol-2-yl)-pyridine 2,6-dicarboxylate,
Dimethyl 4-(1-pyrrolidinyl)-pyridine 2,6-dicarboxylate,
Dimethyl 4-(2-methyl-1-piperidinyl)-pyridine 2,6-dicarboxylate,
Dimethyl 4-(4-morpholinyl)-pyridine 2,6-dicarboxylate,
Diethyl 4-methylpyridine 2,6-dicarboxylate,
Diethyl 4-(hydroxymethyl)-pyridine 2,6-dicarboxylate,
Diethyl 4-ethylpyridine 2,6-dicarboxylate,
Diethyl 4-tert-butylpyridine 2,6-dicarboxylate,
Diethyl 4-(2-carboxyethyl)-pyridine 2,6-dicarboxylate,
Diethyl 4-(2-furanyl)-pyridine 2,6-dicarboxylate,
Diethyl 4-(4-methoxyphenyl)-pyridine 2,6-dicarboxylate,
Diethyl 4-(4-cyanophenyl)-pyridine 2,6-dicarboxylate,
Diethyl 4-(4-chlorophenyl)-pyridine 2,6-dicarboxylate,
Diethyl 4-(2-thienyl)-pyridine 2,6-dicarboxylate,
Diethyl 4-(3-thienyl)-pyridine 2,6-dicarboxylate,
Diethyl 4-[4-(1,1-dimethylethyl)phenyl]-pyridine 2,6-dicarboxylate,
Diethyl 4-(1-piperazinyl)-pyridine 2,6-dicarboxylate,
Diethyl 4-(4-morpholinyl)-pyridine 2,6-dicarboxylate,
Diethyl 4-(1-piperazinyl)-pyridine 2,6-dicarboxylate,
Diethyl 4-(9-anthracenyl)-pyridine 2,6-dicarboxylate,
Diethyl 4-(5-chloro-2-methoxyphenyl)-pyridine 2,6-dicarboxylate,
Diethyl 4-(5-chloro-2-ethoxyphenyl)-pyridine 2,6-dicarboxylate,
Diethyl 4-(2,4,6-trimethoxyphenyl)-pyridine 2,6-dicarboxylate,
Diethyl 4-(3-bromo-2,4,6-trimethoxyphenyl)-pyridine 2,6-dicarboxylate,
Diethyl 4-(3-hexyl-2-thienyl)-pyridine 2,6-dicarboxylate,
Di-tert-butyl 4-(bromomethyl)-pyridine 2,6-dicarboxylate,
Di-tert-butyl 4-(pyridine-4-yl)-pyridine 2,6-dicarboxylate,
Dimethyl 4-(4-hydroxy-2,6-dimethoxyphenyl)pyridine-2,6-dicarboxylate,
Dimethyl 4-(3,5-bis(trifluoromethyl)phenyl)pyridine-2,6-dicarboxylate,
Dimethyl 4-(3,4-dimethoxy-phenyl)-pyridine 2,6-dicarboxylate,
Dimethyl 4-(3-hydroxypropyl)pyridine-2,6-dicarboxylate,
Diethyl 4-phenyl-pyridine 2,6-dicarboxylate,
Diethyl 4-(4-ethylphenyl)-pyridine 2,6-dicarboxylate,
Dimethyl 4-(4-phenyl-1H-1,2,3-triazol-1-yl)pyridine-2,6-dicarboxylate, and
2-(2,6-bis(methoxycarbonyl)pyridin-4-yl)acetic acid.

2. The compound of formula I according to claim 1, wherein
R¹ is C₁-C₈-alkyl, C₃-C₈-cycloalkyl, phenyl, naphthyl or 5- or 6-membered heteroaryl containing at least one hetero atom selected from nitrogen, oxygen and sulfur, which is optionally substituted with one or more substituents selected from the group consisting of C₁-C₈-alkyl, C₂-C₈-alkenyl, C₂-C₈-alkynyl, C₁-C₈-alkoxy, nitro, cyano, halogen, hydroxyl, carboxyl, halo-C₁-C₈-alkyl, halo-C₁-C₈-alkoxy and phenyl; and
R² is C₁-C₈-alkyl or benzyl;
or racemate, enantiomer, diastereomer, mixture thereof, or pharmaceutically acceptable salt thereof.

3. The compound of formula I according to claim 1 or 2, wherein
R¹ is C₁-C₈-alkyl substituted with one or more substituents selected from the group consisting of C₂-C₈-alkenyl, C₂-C₈-alkynyl, C₁-C₈-alkoxy, nitro, cyano, halogen, carboxyl, halo- C₁-C₈-alkyl, halo- C₁-C₈-alkoxy and phenyl; and
R² is C₁-C₄-alkyl, or benzyl;
or racemate, enantiomer, diastereomer, mixture thereof, or pharmaceutically acceptable salt thereof.

4. The compound of formula I according to claim 1 or 2, wherein
R¹ is C₃-C₈-cycloalkyl substituted with one or more substituents selected from the group C₂-C₈-alkenyl, C₂-C₈-alkynyl, C₁-C₈-alkoxy, nitro, cyano, halogen, carboxyl, halo- C₁-C₈-alkyl, halo- C₁-C₈-alkoxy and pheny; and
R² is C₁-C₄-alkyl, or benzyl;
or racemate, enantiomer, diastereomer, mixture thereof, or pharmaceutically acceptable salt thereof.

5. The compound of formula I according to claim 1 or 2, wherein
R¹ is phenyl substituted with one or more substituents selected from the group methyl, ethyl, propyl, isopropyl, ethenyl, 2-propenyl, ethynyl, propargyl, trifluoromethyl, trifluoromethoxy, hydroxyl, carboxyl, nitro;
R² is C₁-C₄-alkyl, or benzyl; and
or racemate, enantiomer, diastereomer, mixture thereof, or pharmaceutically acceptable salt thereof.

6. The compound of formula I according to claim 1 or 2, wherein
R¹ is 5- or 6-membered heteroaryl containing at least one hetero atom selected from nitrogen, oxygen and sulfur substituted with one or more substituents selected from the group C₂-C₈-alkenyl, C₂-C₈-alkynyl, C₁-C₈-alkoxy, nitro, cyano, halogen, carboxyl, halo- C₁-C₈-alkyl, halo- C₁-C₈-alkoxy and phenyl; and
R² is C₁-C₄-alkyl, or benzyl;
or racemate, enantiomer, diastereomer, mixture thereof, or pharmaceutically acceptable salt thereof.

7. The compound of formula I according to claim 1 or 2, wherein
R¹ is 5- or 6-membered heterocyclyl containing at least one hetero atom selected from nitrogen, oxygen and sulfur substituted with one or more substituents selected from the group C₂-C₈-alkenyl, C₂-C₈-alkynyl, C₁-C₈-alkoxy, nitro, cyano, halogen, carboxyl, halo- C₁-C₈-alkyl, halo-C₁-C₈-alkoxy and phenyl; and
R² is C₁-C₄-alkyl, or benzyl;
or racemate, enantiomer, diastereomer, mixture thereof, or pharmaceutically acceptable salt thereof.

8. The compound of formula I according to any one of claims 1 to 7, wherein
R² is ethyl or benzyl;
or racemate, enantiomer, diastereomer, mixture thereof, or pharmaceutically acceptable salt thereof.

9. The compound of formula I according to any one of claims 1 to 8 selected from the group consisting of: 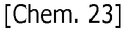 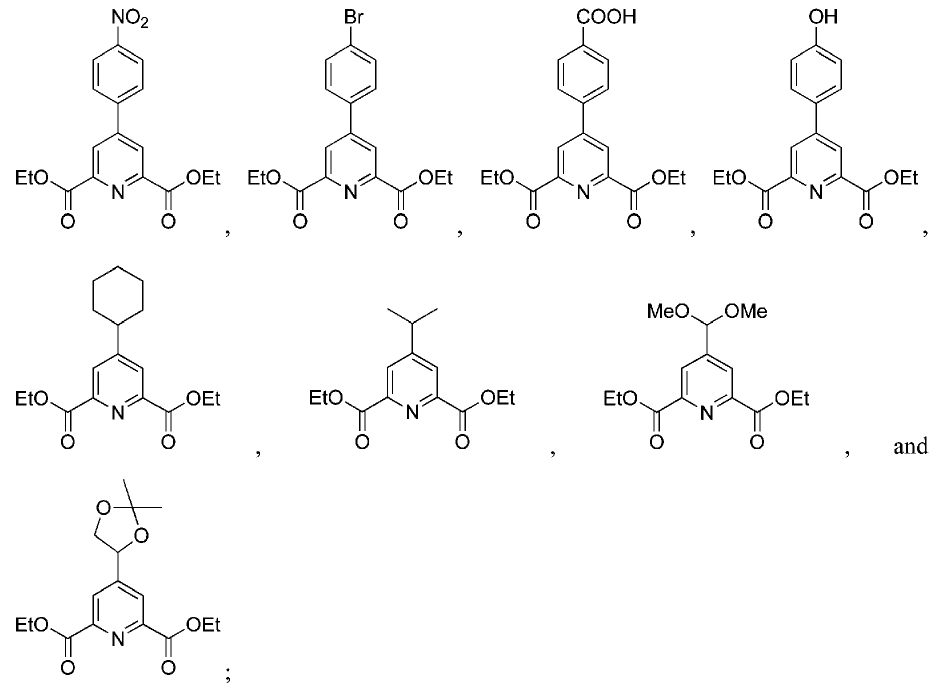 or racemate, enantiomer, diastereomer, mixture thereof, or pharmaceutically acceptable salt thereof.

10. A process for preparing compound of formula I: 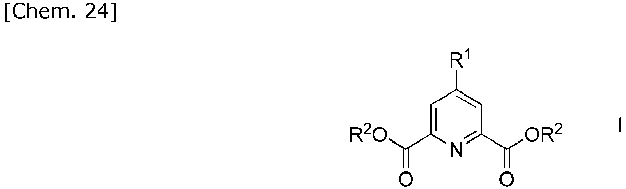 wherein
R¹ and R² are as defined in claim 1;
which process comprises reacting compound of formula II: 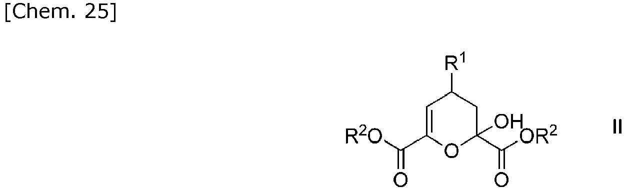 wherein
R¹ and R² are as defined in claim 1;
with NH₄OAc in a solvent.

11. A process for preparing compound of formula I according to claim 10, wherein the solvent is CH₃CN.

12. A pharmaceutical composition comprising a compound according to any one of claims 1 to 9, and a pharmaceutically acceptable adjuvant.

13. The pharmaceutical composition according to claim 12 for use in the treatment of proliferative diseases.

## Patentansprüche

1. Verbindung, umfassend die Struktur nach Formel I, wobei 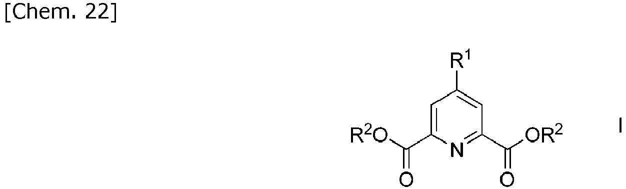
R¹ C₁-C₁₈-Alkyl, C₃-C₁₈-Cycloalkyl, Aryl, C₃-C₁₈-Heterocycloalkyl oder Heteroaryl ist, das gegebenenfalls mit einem oder mehreren Substituenten substituiert ist, ausgewählt aus der Gruppe bestehend aus C₁-C₁₈-Alkyl, C₂-C₁₈-Alkenyl, C₂-C₁₈-Alkinyl, C₁-C₁₈-Alkoxy, Nitro, Cyano, Halogen, Hydroxyl, Carboxyl, Halogen-C₁-C₁₈-alkyl, Halogen-C₁-C₁₈-alkoxy und Phenyl; und
R² C₁-C₁₈-Alkyl oder Benzyl ist;
oder Racemate, Enantiomere, Diastereomere, Gemische der Verbindung oder pharmazeutisch annehmbare Salze davon, außer
Dimethyl-4-methylpyridin-2,6-dicarboxylat,
Dimethyl-4-(hydroxymethyl)-pyridin-2,6-dicarboxylat,
Dimethyl-4-ethylpyridin-2,6-dicarboxylat,
Dimethyl-4-isopropylpyridin-2,6-dicarboxylat,
Dimethyl-4-(hydroxyethyl)-pyridin-2,6-dicarboxylat,
Dimethyl-4-(chloromethyl)pyridin-2,6-dicarboxylat,
Dimethyl-4-(iodomethyl)pyridin-2,6-dicarboxylat,
Dimethyl-4-(bromomethyl)pyridin-2,6-dicarboxylat,
Dimethyl-4-(dimethoxymethyl)pyridin-2,6-dicarboxylat,
Dimethyl-4-(hydroxyethyl)-pyridin-2,6-dicarboxylat,
Dimethyl-4-phenyl-pyridin-2,6-dicarboxylat,
Dimethyl-4-(3-bromopropyl)-pyridin-2,6-dicarboxylat,
Dimethyl-4-(trifluoromethyl)-pyridin-2,6-dicarboxylat,
Dimethyl-4-(3-methylphenyl)-pyridin-2,6-dicarboxylat,
Dimethyl-4-cyclohexyl-pyridin-2,6-dicarboxylat,
Dimethyl-4-(4-methylphenyl)-pyridin-2,6-dicarboxylat,
Dimethyl-4-(4-hydroxyphenyl)-pyridin-2,6-dicarboxylat,
Dimethyl-4-(2-methylphenyl)-pyridin-2,6-dicarboxylat,
Dimethyl-4-(3-chlorophenyl)-pyridin-2,6-dicarboxylat,
Dimethyl-4-naphthalenyl-pyridin-2,6-dicarboxylat,
Dimethyl-4-(4-methoxylphenyl)-pyridin-2,6-dicarboxylat,
Dimethyl-4-(4-fluorophenyl)-pyridin-2,6-dicarboxylat,
Dimethyl-4-(4-nitrophenyl)-pyridin-2,6-dicarboxylat,
Dimethyl-4-(4-hydroxy-2-methoxylphenyl)-pyridin-2,6-dicarboxylat,
Dimethyl-4-(2,4-dimethoxylphenyl)-pyridin-2,6-dicarboxylat,
Dimethyl-4-[(4-octyloxyl)phenyl]-pyridin-2,6-dicarboxylat,
Dimethyl-4-benzo[b]thien-2-yl-pyridin-2,6-dicarboxylat,
Dimethyl-4-[(4-octadecyloxyl)phenyl]-pyridin-2,6-dicarboxylat,
Dimethyl-4-(1-methyl-1H-indol-2-yl)-pyridin-2,6-dicarboxylat,
Dimethyl-4-(1-pyrrolidinyl)-pyridin-2,6-dicarboxylat,
Dimethyl-4-(2-methyl-1-piperidinyl)-pyridin-2,6-dicarboxylat,
Dimethyl-4-(4-morpholinyl)-pyridin-2,6-dicarboxylat,
Diethyl-4-methylpyridin-2,6-dicarboxylat,
Diethyl-4-(hydroxymethyl)-pyridin-2,6-dicarboxylat,
Diethyl-4-ethylpyridin-2,6-dicarboxylat,
Diethyl-4-tert-butylpyridin-2,6-dicarboxylat,
Diethyl-4-(2-carboxyethyl)-pyridin-2,6-dicarboxylat,
Diethyl-4-(2-furanyl)-pyridin-2,6-dicarboxylat,
Diethyl-4-(4-methoxyphenyl)-pyridin-2,6-dicarboxylat,
Diethyl-4-(4-cyanophenyl)-pyridin-2,6-dicarboxylat,
Diethyl-4-(4-chlorophenyl)-pyridin-2,6-dicarboxylat,
Diethyl-4-(2-thienyl)-pyridin-2,6-dicarboxylat,
Diethyl-4-(3-thienyl)-pyridin-2,6-dicarboxylat,
Diethyl-4-[4-(1,1-dimethylethyl)phenyl]-pyridin-2,6-dicarboxylat,
Diethyl-4-(1-piperazinyl)-pyridin-2,6-dicarboxylat,
Diethyl-4-(4-morpholinyl)-pyridin-2,6-dicarboxylat,
Diethyl-4-(1-piperazinyl)-pyridin-2,6-dicarboxylat,
Diethyl-4-(9-anthracenyl)-pyridin-2,6-dicarboxylat,
Diethyl-4-(5-chloro-2-methoxyphenyl)-pyridin-2,6-dicarboxylat,
Diethyl-4-(5-chloro-2-ethoxyphenyl)-pyridin-2,6-dicarboxylat,
Diethyl-4-(2,4,6-trimethoxyphenyl)-pyridin-2,6-dicarboxylat,
Diethyl-4-(3-bromo-2,4,6-trimethoxyphenyl)-pyridin-2,6-dicarboxylat,
Diethyl-4-(3-hexyl-2-thienyl)-pyridin-2,6-dicarboxylat,
Di-tert-butyl-4-(bromomethyl)-pyridin-2,6-dicarboxylat,
Di-tert-butyl-4-(pyridin-4-yl)-pyridin-2,6-dicarboxylat,
Dimethyl-4-(4-hydroxy-2,6-dimethoxyphenyl)pyridin-2,6-dicarboxylat,
Dimethyl-4-(3,5-bis(trifluoromethyl)phenyl)pyridin-2,6-dicarboxylat,
Dimethyl-4-(3,4-dimethoxy-phenyl)-pyridin-2,6-dicarboxylat,
Dimethyl-4-(3-hydroxypropyl)pyridin-2,6-dicarboxylat,
Diethyl-4-phenyl-pyridin-2,6-dicarboxylat,
Diethyl-4-(4-ethylphenyl)-pyridin-2,6-dicarboxylat,
Dimethyl-4-(4-phenyl-1H-1,2,3-triazol-1-yl)pyridin-2,6-dicarboxylat und 2-(2,6-bis(Methoxycarbonyl)pyridin-4-yl)essigsäure.

2. Verbindung von Formel I nach Anspruch 1, wobei
R¹ C₁-C₈-Alkyl, C₃-C₈-Cycloalkyl, Phenyl, Naphthyl oder 5- oder 6-gliedriges Heteroaryl ist, das zumindest ein Heteroatom enthält, ausgewählt aus Stickstoff, Sauerstoff und Schwefel, das gegebenenfalls mit einem oder mehreren Substituenten substituiert ist, ausgewählt aus der Gruppe bestehend aus C₁-C₈-Alkyl, C₂-C₈-Alkenyl, C₂-C₈-Alkinyl, C₁-C₈-Alkoxy, Nitro, Cyano, Halogen, Hydroxyl, Carboxyl, Halogen-C₁-C₈-Alkyl, Halogen-C₁-C₈-Alkoxy und Phenyl; und
R² C₁-C₈-Alkyl oder Benzyl ist;
oder Racemat, Enantiomer, Diastereomer, ein Gemisch davon oder ein pharmazeutisch annehmbares Salz davon.

3. Verbindung von Formel I nach Anspruch 1 oder 2, wobei
R¹ C₁-C₈-Alkyl ist, substituiert mit einem oder mehreren Substituenten, ausgewählt aus der Gruppe bestehend aus C₂-C₈-Alkenyl, C₂-C₈-Alkinyl, C₁-C₈-Alkoxy, Nitro, Cyano, Halogen, Carboxyl, Halogen-C₁-C₈-alkyl, Halogen-C₁-C₈-alkoxy und Phenyl; und
R² C₁-C₄-Alkyl oder Benzyl ist;
oder Racemat, Enantiomer, Diastereomer, ein Gemisch davon oder ein pharmazeutisch annehmbares Salz davon.

4. Verbindung von Formel I nach Anspruch 1 oder 2, wobei
R¹ C₃-C₈-Cycloalkyl ist, substituiert mit einem oder mehreren Substituenten, ausgewählt aus der Gruppe von C₂-C₈-Alkenyl, C₂-C₈-Alkinyl, C₁-C₈-Alkoxy, Nitro, Cyano, Halogen, Carboxyl, Halogen-C₁-C₈-alkyl, Halogen-C₁-C₈-alkoxy und Phenyl; und
R² C₁-C₄-Alkyl oder Benzyl ist;
oder Racemat, Enantiomer, Diastereomer, ein Gemisch davon oder ein pharmazeutisch annehmbares Salz davon.

5. Verbindung von Formel I nach Anspruch 1 oder 2, wobei
R¹ Phenyl ist, substituiert mit einem oder mehreren Substituenten, ausgewählt aus der Gruppe von Methyl, Ethyl, Propyl, Isopropyl, Ethenyl, 2-Propenyl, Ethinyl, Propargyl, Trifluoromethyl, Trifluoromethoxy, Hydroxyl, Carboxyl, Nitro;
R² C₁-C₄-Alkyl oder Benzyl ist; und
oder Racemat, Enantiomer, Diastereomer, ein Gemisch davon oder ein pharmazeutisch annehmbares Salz davon.

6. Verbindung von Formel I nach Anspruch 1 oder 2, wobei
R¹ 5- oder 6-gliedriges Heteroaryl ist, enthaltend zumindest ein Heteroatom, ausgewählt aus Stickstoff, Sauerstoff und Schwefel, substituiert mit einem oder mehreren Substituenten, ausgewählt aus der Gruppe von C₂-C₈-Alkenyl, C₂-C₈-Alkinyl, C₁-C₈-Alkoxy, Nitro, Cyano, Halogen, Carboxyl, Halogen-C₁-C₈-alkyl, Halogen-C₁-C₈-alkoxy und Phenyl; und
R² C₁-C₄-Alkyl oder Benzyl ist;
oder Racemat, Enantiomer, Diastereomer, ein Gemisch davon oder ein pharmazeutisch annehmbares Salz davon.

7. Verbindung von Formel I nach Anspruch 1 oder 2, wobei
R¹ 5- oder 6-gliedriges Heterocyclyl ist, enthaltend zumindest ein Heteroatom, ausgewählt aus Stickstoff, Sauerstoff und Schwefel, substituiert mit einem oder mehreren Substituenten, ausgewählt aus der Gruppe von C₂-C₈-Alkenyl, C₂-C₈-Alkinyl, C₁-C₈-Alkoxy, Nitro, Cyano, Halogen, Carboxyl, Halogen-C₁-C₈-alkyl, Halogen-C₁-C₈-alkoxy und Phenyl; und
R² C₁-C₄-Alkyl oder Benzyl ist;
oder Racemat, Enantiomer, Diastereomer, ein Gemisch davon oder ein pharmazeutisch annehmbares Salz davon.

8. Verbindung von Formel I nach einem der Ansprüche 1 bis 7, wobei R² Ethyl oder Benzyl ist;
oder Racemat, Enantiomer, Diastereomer, ein Gemisch davon oder ein pharmazeutisch annehmbares Salz davon.

9. Verbindung von Formel I nach einem der Ansprüche 1 bis 8, ausgewählt aus der Gruppe bestehend aus: 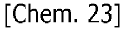 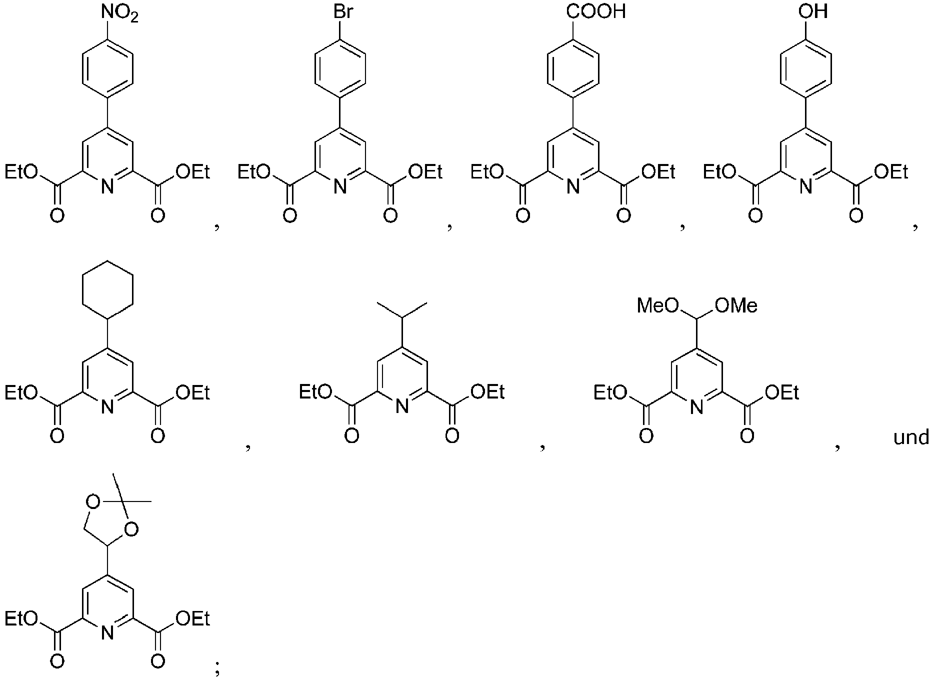 oder Racemat, Enantiomer, Diastereomer, ein Gemisch davon oder ein pharmazeutisch annehmbares Salz davon.

10. Verfahren zum Herstellen der Verbindung von Formel I: 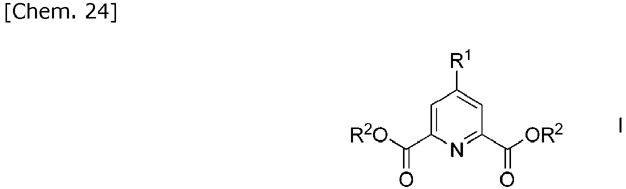 wobei
R¹ und R² wie in Anspruch 1 definiert sind;
wobei das Verfahren ein Umsetzen der Verbindung von Formel II umfasst: 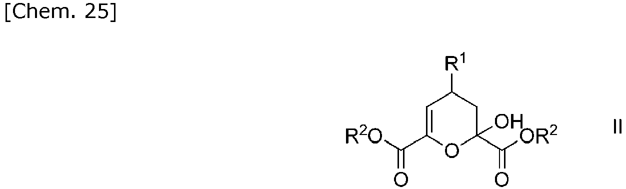 wobei
R¹ und R² wie in Anspruch 1 definiert sind;
mit NH₄OAc in einem Lösungsmittel.

11. Verfahren zum Herstellen der Verbindung von Formel I nach Anspruch 10, wobei das Lösungsmittel CH₃CN ist.

12. Pharmazeutische Zusammensetzung, umfassend eine Verbindung nach einem der Ansprüche 1 bis 9 und einen pharmazeutisch annehmbaren Adjuvans.

13. Pharmazeutische Zusammensetzung nach Anspruch 12 zur Verwendung bei der Therapie von proliferativen Erkrankungen.

## Revendications

1. Composé comprenant la structure selon la formule I, dans laquelle 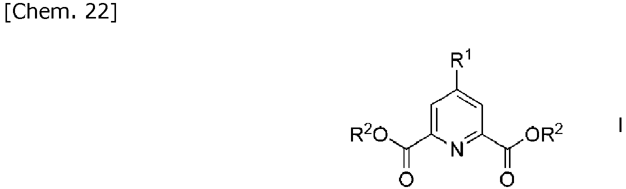
R¹ représente un groupe (C₁ à C₁₈)alkyle, (C₃ à C₁₈)cycloalkyle, aryle, (C₃ à C₁₈)hétérocycloalkyle ou hétéroaryle, qui est éventuellement substitué par un ou plusieurs substituants choisis dans le groupe constitué par un groupe (C₁ à C₁₈)alkyle, (C₂ à C₁₈)alcényle, (C₂ à C₁₈)alcynyle, (C₁ à C₁₈)alcoxy, nitro, cyano, halogéno, hydroxy, carboxy, halogéno-(C₁ à C₁₈)alkyle, halogéno-(C₁ à C₁₈)alcoxy et phényle ; et
R² représente un groupe (C₁ à C₁₈)alkyle ou benzyle ;
ou racémiques, énantiomères, diastéréoisomères, ou mélanges du composé, ou sels pharmaceutiquement acceptables de ceux-ci, à l'exception du
4-méthylpyridine-2,6-dicarboxylate de diméthyle,
4-(hydroxyméthyl)-pyridine-2,6-dicarboxylate de diméthyle,
4-éthylpyridine-2,6-dicarboxylate de diméthyle,
4-isopropylpyridine-2,6-dicarboxylate de diméthyle,
4-(hydroxyéthyl)-pyridine-2,6-dicarboxylate de diméthyle,
4-(chlorométhyl)pyridine-2,6-dicarboxylate de diméthyle,
4-(iodométhyl)pyridine-2,6-dicarboxylate de diméthyle,
4-(bromométhyl)pyridine-2,6-dicarboxylate de diméthyle,
4-(diméthoxyméthyl)pyridine-2,6-dicarboxylate de diméthyle,
4-(hydroxyéthyl)-pyridine-2,6-dicarboxylate de diméthyle,
4-phényl-pyridine-2,6-dicarboxylate de diméthyle,
4-(3-bromopropyl)-pyridine-2,6-dicarboxylate de diméthyle,
4-(trifluorométhyl)-pyridine-2,6-dicarboxylate de diméthyle,
4-(3-méthylphényl)-pyridine-2,6-dicarboxylate de diméthyle,
4-cyclohexyl-pyridine-2,6-dicarboxylate de diméthyle,
4-(4-méthylphényl)-pyridine-2,6-dicarboxylate de diméthyle,
4-(4-hydroxyphényl)-pyridine-2,6-dicarboxylate de diméthyle,
4-(2-méthylphényl)-pyridine-2,6-dicarboxylate de diméthyle,
4-(3-chlorophényl)-pyridine-2,6-dicarboxylate de diméthyle,
4-naphtalényl-pyridine-2,6-dicarboxylate de diméthyle,
4-(4-méthoxylphényl)-pyridine-2,6-dicarboxylate de diméthyle,
4-(4-fluorophényl)-pyridine-2,6-dicarboxylate de diméthyle,
4-(4-nitrophényl)-pyridine-2,6-dicarboxylate de diméthyle,
4-(4-hydroxy-2-méthoxylphényl)-pyridine-2,6-dicarboxylate de diméthyle,
4-(2,4-diméthoxylphényl)-pyridine-2,6-dicarboxylate de diméthyle,
4-[(4-octyloxyl)phényl]-pyridine-2,6-dicarboxylate de diméthyle,
4-benzo[b]thién-2-yl-pyridine-2,6-dicarboxylate de diméthyle,
4-[(4-octadécyloxyl)phényl]-pyridine-2,6-dicarboxylate de diméthyle,
4-(1-méthyl-1H-indol-2-yl)-pyridine-2,6-dicarboxylate de diméthyle,
4-(1-pyrrolidinyl)-pyridine-2,6-dicarboxylate de diméthyle,
4-(2-méthyl-1-pipéridinyl)-pyridine-2,6-dicarboxylate de diméthyle,
4-(4-morpholinyl)-pyridine-2,6-dicarboxylate de diméthyle,
4-méthylpyridine-2,6-dicarboxylate de diéthyle,
4-(hydroxyméthyl)-pyridine-2,6-dicarboxylate de diéthyle,
4-éthylpyridine-2,6-dicarboxylate de diéthyle,
4-tert-butylpyridine-2,6-dicarboxylate de diéthyle,
4-(2-carboxyéthyl)-pyridine-2,6-dicarboxylate de diéthyle,
4-(2-furanyl)-pyridine-2,6-dicarboxylate de diéthyle,
4-(4-méthoxyphényl)-pyridine-2,6-dicarboxylate de diéthyle,
4-(4-cyanophényl)-pyridine-2,6-dicarboxylate de diéthyle,
4-(4-chlorophényl)-pyridine-2,6-dicarboxylate de diéthyle,
4-(2-thiényl)-pyridine-2,6-dicarboxylate de diéthyle,
4-(3-thiényl)-pyridine-2,6-dicarboxylate de diéthyle,
4-[4-(1,1-diméthyléthyl)phényl]-pyridine-2,6-dicarboxylate de diéthyle,
4-(1-pipérazinyl)-pyridine-2,6-dicarboxylate de diéthyle,
4-(4-morpholinyl)-pyridine-2,6-dicarboxylate de diéthyle,
4-(1-pipérazinyl)-pyridine-2,6-dicarboxylate de diéthyle,
4-(9-anthracényl)-pyridine-2,6-dicarboxylate de diéthyle,
4-(5-chloro-2-méthoxyphényl)-pyridine-2,6-dicarboxylate de diéthyle,
4-(5-chloro-2-éthoxyphényl)-pyridine-2,6-dicarboxylate de diéthyle,
4-(2,4,6-triméthoxyphényl)-pyridine-2,6-dicarboxylate de diéthyle,
4-(3-bromo-2,4,6-triméthoxyphényl)-pyridine-2,6-dicarboxylate de diéthyle,
4-(3-hexyl-2-thiényl)-pyridine-2,6-dicarboxylate de diéthyle,
4-(bromométhyl)-pyridine-2,6-dicarboxylate de di-tert-butyle,
4-(pyridine-4-yl)-pyridine-2,6-dicarboxylate de di-tert-butyle,
4-(4-hydroxy-2,6-diméthoxyphényl)pyridine-2,6-dicarboxylate de diméthyle,
4-(3,5-bis(trifluorométhyl)phényl)pyridine-2,6-dicarboxylate de diméthyle,
4-(3,4-diméthoxy-phényl)-pyridine-2,6-dicarboxylate de diméthyle,
4-(3-hydroxypropyl)pyridine-2,6-dicarboxylate de diméthyle,
4-phényl-pyridine-2,6-dicarboxylate de diéthyle,
4-(4-éthylphényl)-pyridine-2,6-dicarboxylate de diéthyle,
4-(4-phényl-1H-1,2,3-triazol-1-yl)pyridine-2,6-dicarboxylate de diméthyle, et de l'acide 2-(2,6-bis(méthoxycarbonyl)pyridin-4-yl)acétique.

2. Composé de formule I selon la revendication 1, dans laquelle
R¹ représente un groupe (C₁ à C₈)alkyle, (C₃ à C₈)cycloalkyle, phényle, naphtyle ou hétéroaryle à 5 ou 6 chaînons contenant au moins un hétéroatome choisi parmi un atome d'azote, d'oxygène et de soufre, qui est éventuellement substitué par un ou plusieurs substituants choisis dans le groupe constitué par un groupe (C₁ à C₈)alkyle, (C₂ à C₈)alcényle, (C₂ à C₈)alcynyle, (C₁ à C₈)alcoxy, nitro, cyano, halogéno, hydroxy, carboxy, halogéno-(C₁ à C₈)alkyle, halogéno-(C₁ à C₈)alcoxy et phényle ; et
R² représente un groupe (C₁ à C₈)alkyle ou benzyle ;
ou racémique, énantiomère, diastéréoisomère, mélange de celui-ci, ou sel pharmaceutiquement acceptable de celui-ci.

3. Composé de formule I selon la revendication 1 ou 2, dans laquelle
R¹ représente un groupe (C₁ à C₈)alkyle substitué par un ou plusieurs substituants choisis dans le groupe constitué par un groupe (C₂ à C₈)alcényle, (C₂ à C₈)alcynyle, (C₁ à C₈)alcoxy, nitro, cyano, halogéno, carboxy, halogéno-(C₁ à C₈)alkyle, halogéno-(C₁ à C₈)alcoxy et phényle ; et
R² représente un groupe (C₁ à C₄)alkyle ou benzyle ;
ou racémique, énantiomère, diastéréoisomère, mélange de celui-ci, ou sel pharmaceutiquement acceptable de celui-ci.

4. Composé de formule I selon la revendication 1 ou 2, dans laquelle
R¹ représente un groupe (C₃ à C₈)cycloalkyle substitué par un ou plusieurs substituants choisis dans le groupe constitué par un groupe (C₂ à C₈)alcényle, (C₂ à C₈)alcynyle, (C₁ à C₈)alcoxy, nitro, cyano, halogéno, carboxy, halogéno-(C₁ à C₈)alkyle, halogéno-(C₁ à C₈)alcoxy et phényle ; et
R² représente un groupe (C₁ à C₄)alkyle ou benzyle ;
ou racémique, énantiomère, diastéréoisomère, mélange de celui-ci, ou sel pharmaceutiquement acceptable de celui-ci.

5. Composé de formule I selon la revendication 1 ou 2, dans laquelle
R¹ représente un groupe phényle substitué par un ou plusieurs substituants choisis parmi le groupe méthyle, éthyle, propyle, isopropyle, éthényle, 2-propényle, éthynyle, propargyle, trifluorométhyle, trifluorométhoxy, hydroxy, carboxy, nitro ;
R² représente un groupe (C₁ à C₄)alkyle ou benzyle ; et
ou racémique, énantiomère, diastéréoisomère, mélange de celui-ci, ou sel pharmaceutiquement acceptable de celui-ci.

6. Composé de formule I selon la revendication 1 ou 2, dans laquelle
R¹ représente un groupe hétéroaryle à 5 ou 6 chaînons contenant au moins un hétéroatome choisi parmi un atome d'azote, d'oxygène et de soufre substitué par un ou plusieurs substituants choisis parmi le groupe (C₂ à C₈)alcényle, (C₂ à C₈)alcynyle, (C₁ à C₈)alcoxy, nitro, cyano, halogéno, carboxy, halogéno-(C₁ à C₈)alkyle, halogéno-(C₁ à C₈)alcoxy et phényle ; et
R² représente un groupe (C₁ à C₄)alkyle ou benzyle ;
ou racémique, énantiomère, diastéréoisomère, mélange de celui-ci, ou sel pharmaceutiquement acceptable de celui-ci.

7. Composé de formule I selon la revendication 1 ou 2, dans laquelle
R¹ représente un groupe hétérocyclyle à 5 ou 6 chaînons contenant au moins un hétéroatome choisi parmi un atome d'azote, d'oxygène et de soufre substitué par un ou plusieurs substituants choisis parmi le groupe (C₂ à C₈)alcényle, (C₂ à C₈)alcynyle, (C₁ à C₈)alcoxy, nitro, cyano, halogéno, carboxy, halogéno-(C₁ à C₈)alkyle, halogéno-(C₁ à C₈)alcoxy et phényle ; et
R² représente un groupe (C₁ à C₄)alkyle ou benzyle ;
ou racémique, énantiomère, diastéréoisomère, mélange de celui-ci, ou sel pharmaceutiquement acceptable de celui-ci.

8. Composé de formule I selon l'une quelconque des revendications 1 à 7, dans laquelle
R² représente un groupe éthyle ou benzyle ;
ou racémique, énantiomère, diastéréoisomère, mélange de celui-ci, ou sel pharmaceutiquement acceptable de celui-ci.

9. Composé de formule I selon l'une quelconque des revendications 1 à 8 choisi dans le groupe constitué par : 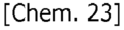 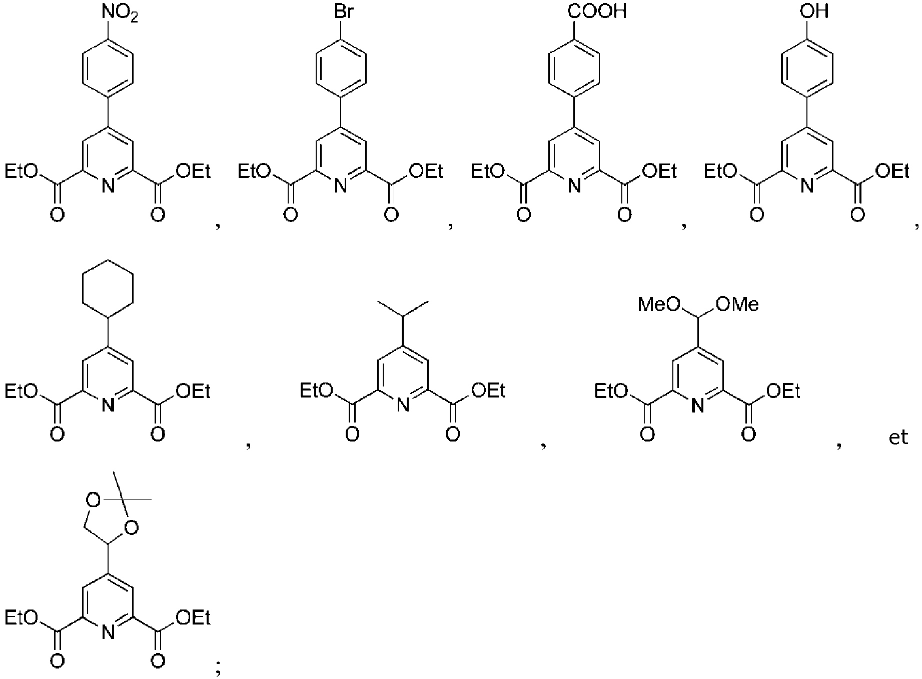 ou racémique, énantiomère, diastéréoisomère, mélange de celui-ci, ou sel pharmaceutiquement acceptable de celui-ci.

10. Procédé de préparation du composé de formule I : 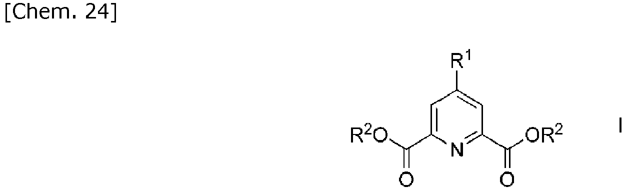 dans laquelle
R¹ et R² sont tels que définis dans la revendication 1 ;
lequel procédé comprend la mise en réaction du composé de formule II : 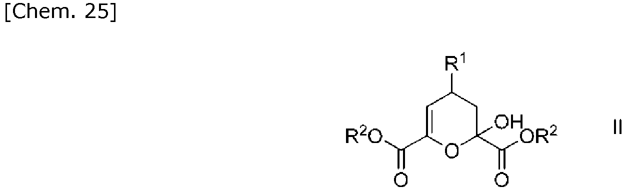 dans laquelle
R¹ et R² sont tels que définis dans la revendication 1 ;
avec NH₄OAc dans un solvant.

11. Procédé de préparation d'un composé de formule I selon la revendication 10, dans lequel le solvant est CH₃CN.

12. Composition pharmaceutique comprenant un composé selon l'une quelconque des revendications 1 à 9, et un adjuvant pharmaceutiquement acceptable.

13. Composition pharmaceutique selon la revendication 12 pour utilisation dans le traitement de maladies prolifératives.
